(19) Europäisches Patentamt
European Patent Office
Office européen des brevets

(11) Veröffentlichungsnummer: **0 136 661**
**B1**

(12) # EUROPÄISCHE PATENTSCHRIFT

(45) Veröffentlichungstag der Patentschrift: **07.01.88**

(51) Int. Cl.⁴: **C 07 D 493/08**, A 61 K 31/40 // (C07D493/08, 319:00, 311:00)

(21) Anmeldenummer: **84111550.4**

(22) Anmeldetag: **27.09.84**

(54) **N- (2,10-Dioxa-tricyclo-(5,3,1,03,8)-undecan-5-yl)-tryptamin-Derivate.**

(30) Priorität: **01.10.83 DE 3335826**

(43) Veröffentlichungstag der Anmeldung: **10.04.85 Patentblatt 85/15**

(45) Bekanntmachung des Hinweises auf die Patenterteilung: **07.01.88 Patentblatt 88/1**

(84) Benannte Vertragsstaaten: **BE CH DE FR GB IT LI NL**

(56) Entgegenhaltungen: **DE - A - 2 129 507**

(73) Patentinhaber: **Kali-Chemie Pharma GmbH, Hans-Böckler-Allee 20 Postfach 220, D-3000 Hannover 1 (DE)**

(72) Erfinder: **Thies, Peter Willibrord, Dipl.-Chem. Dr. phil, Ihmeplatz 6, D-3000 Hannover 91 (DE)**
Erfinder: **David, Samuel, Dipl.-Chem. Dr. rer. nat., Gollstrasse 9, D-3000 Hannover 73 (DE)**
Erfinder: **Kühl, Ulrich G., Dr. med. vet., Franzburger Strasse 10, D-3007 Gehrden 1 (DE)**
Erfinder: **Buschmann, Gerd, Dr. med. vet., Matthäikirchstrasse 27, D-3000 Hannover (DE)**
Erfinder: **Flecker, Peter, Dr. Dipl.-Chem., Alte Frankfurter Strasse 44, D-6368 Bad Vilbel 2 (DE)**

(74) Vertreter: **Lauer, Dieter, Dr., c/o Kali-Chemie AG Postfach 220 Hans-Böckler-Allee 20, D-3000 Hannover 1 (DE)**

EP 0 136 661 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft N-(2,10-Dioxa-tricyclo-[5,3,1,$0^{3.8}$]-undecan-5-yl)-tryptamin-Verbindungen und deren Säureadditionssalze sowie diese Verbindungen enthaltende pharmazeutische Zubereitungen und Verfahren zur Herstellung dieser Verbindungen.

Der Erfindung liegt die Aufgabe zugrunde, neue das Herzkreislauf-System günstig beeinflussende Pharmaka zu entwickeln.

Es wurde nun gefunden, dass die erfindungsgemässen N-(2,10-Dioxa-tricyclo-[5,3,1,$0^{3.8}$]-undecan-5-yl)-tryptamin-Verbindungen wertvolle pharmakologische Eigenschaften, insbesondere blutdrucksenkende Wirkungen, besitzen und ein vorteilhaftes Wirkungsprofil mit guter therapeutischer Breite und geringer Toxizität aufweisen. Aufgrund ihrer Wirkungen sind die neuen Verbindungen als Arzneimittel insbesondere zur Behandlung von Bluthochdruck geeignet.

Die vorliegende Erfindung betrifft daher neue Verbindungen der allgemeinen Formel (I)

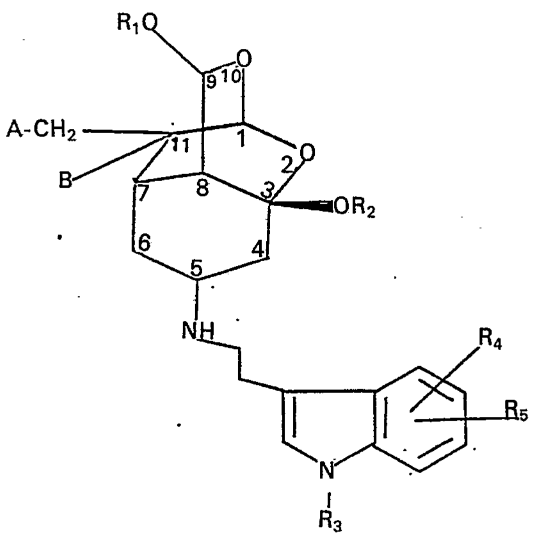

I

worin $R_1$ Wasserstoff, Alkyl mit 1–4 Kohlenstoffatomen oder Benzyl bedeutet,

$R_2$ Alkyl mit 1–4 Kohlenstoffatomen oder Alkanoyl mit 1–4 Kohlenstoffatomen bedeutet,

$R_3$ Wasserstoff oder Alkyl mit 1–4 Kohlenstoffatomen bedeutet,

$R_4$ Wasserstoff, Alkoxy mit 1–4 Kohlenstoffatomen, Benzyloxy oder Hydroxy bedeutet,

$R_5$ Wasserstoff, Alkoxy mit 1–4 Kohlenstoffatomen, Benzyloxy oder Hydroxy bedeutet, und

A und B je Wasserstoff bedeuten oder gemeinsam eine Bindung bilden, und deren Säureadditionssalze.

Die in den Resten $R_1$ bis $R_5$ der Verbindungen der Formel (I) enthaltenen $C_1$–$C_4$-Alkylgruppen können gerade oder verzweigt sein und enthalten insbesondere 1 bis 2 Kohlenstoffatome. Der Rest $R_1$ steht vorzugsweise für $C_1$–$C_4$-Alkyl, insbesondere Methyl. Der Rest $R_2$ steht bevorzugt für $C_1$–$C_4$-Alkyl, z.B. Methyl, Äthyl, n-Propyl, Isopropyl, n-Butyl oder tert.-Butyl, oder für $C_1$–$C_4$-Alkanoyl, z.B. Acetyl. Die Reste $R_3$ bis $R_5$ stehen vorzugsweise für Wasserstoff. Falls $R_3$ $C_1$–$C_4$-Alkyl bedeutet, stellt es vorzugsweise Methyl dar. Falls $R_4$ und/oder $R_5$ $C_1$–$C_4$-Alkoxy bedeuten, stellen sie vorzugsweise Methoxy dar.

In den Verbindungen der Formel (I) können die Substituenten an den Zentren $C_5$, $C_9$ und $C_{11}$ des 2,10-Dioxa-tricyclo-[5,3,1,$0^{3.8}$]-undecan- Gerüstes jeweils in R- oder S-Stellung angeordnet sein, so dass die Verbindungen in mehreren diastereomeren Formen auftreten können. Falls A und B je Wasserstoff bedeuten, ist $C_{11}$ bevorzugt R-konfiguriert. $C_9$ ist bevorzugt R-konfiguriert.

Die vorliegende Erfindung umfasst alle durch Variation der Konfiguration an den Zentren $C_5$, $C_9$ und $C_{11}$ erhältlichen diastereoisomeren Formen der Verbindungen der Formel (I).

Erfindungsgemäss werden die neuen N-(2,10-Dioxa-tricyclo-[5,3,1,$0^{3.8}$]-undecan-5-yl)-tryptamin-Verbindungen der Formel I und deren Säureadditionssalze erhalten, indem man Verbindungen der allgemeinen Formel (II)

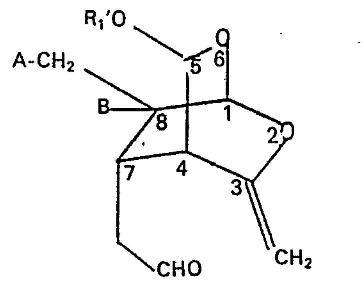

II

worin A und B obige Bedeutung besitzen und $R_1'$ niederes Alkyl oder Benzyl bedeutet, mit einem Amin der allgemeinen Formel (III)

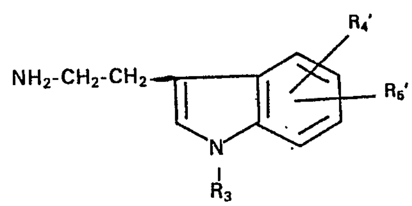

III

worin $R_3$ obige Bedeutung besitzt und $R_4'$ und $R_5'$ die für $R_4$ und $R_5$ genannten Bedeutungen mit Ausnahme von Hydroxy besitzen, und einem nukleophilen Lösungsmittel der allgemeinen Formel (IV)

$R_2OH$ IV

worin $R_2$ obige Bedeutung besitzt, umsetzt, und in den erhaltenen Verbindungen der allgemeinen Formel (Ia)

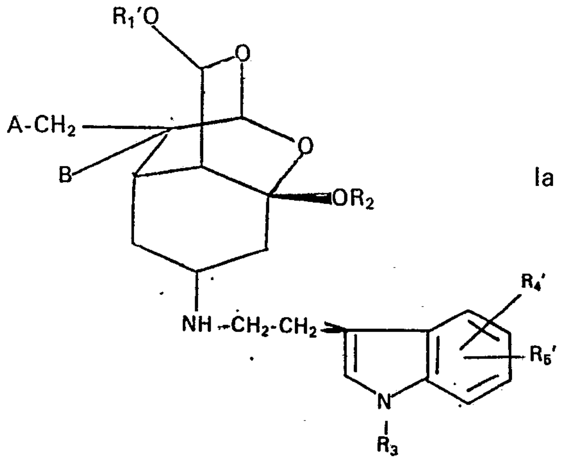

Ia

worin A, B, $R_1'$, $R_2$, $R_3$, $R_4'$ und $R_5'$ obige Bedeutung besitzen, gewünschtenfalls allfällige Benzyloxygruppen hydrogenolytisch zu Hydroxygruppen spaltet, und/oder gegebenenfalls freie Verbindungen der Formel (I) in ihre Säureadditionssalze überführt oder die Säureadditionssalze in die freien Verbindungen der Formel (I) überführt.

Die erfindungsgemässe cyclisierende Umsetzung eines Aldehydes der Formel (II) mit einem Amin der Formel (III) und einem Nukleophil der Formel (IV) kann formal aufgefasst werden als aus zwei Reaktionsschritten bestehend, welche gleichzeitig oder nacheinander ablaufen können. Der erste Schritt stellt eine Kondensationsreaktion des Aldehyds der Formel (II) mit dem Amin der Formel (III) unter Bildung einer Iminfunktion dar. Der zweite Schritt besteht aus einem Angriff des Nukleophils der Formel (IV) an der exocyclischen Enolätherfunktion der Verbindung der Formel (II) mit darauffolgender Cyclisierung unter Addition eines Moleküls der Formel (IV).

Der zweite Schritt wird zweckmässigerweise durch Protonenkatalyse gefördert. Sofern das Nukleophil der Formel (IV) selbst eine Säure darstellt, kann es gleichzeitig die Funktion eines Säurekatalysators erfüllen. Falls als Nukleophil der Formel (IV) ein Alkohol eingesetzt wird, kann es sich als zweckmässig erweisen, dass Amin der Formel (III) in Form eines anorganischen Säureadditionssalzes, beispielsweise eines Hydrochlorids, einzusetzen oder eine katalytische Menge einer anorganischen Säure zuzusetzen.

Erfindungsgemäss wird die Umsetzung des Aldehyds der Formel (II) mit dem Amin (III) in dem nukleophilen Lösungsmittel der Formel (IV) bei Temperaturen zwischen Raumtemperatur und 100 °C, vorzugsweise zwischen 40 und 80 °C, durchgeführt. Als Lösungsmittel kann das nukleophile Lösungsmittel (IV) alleine oder im Gemisch mit weiteren inerten aprotischen organischen Lösungsmitteln, z.B. aromatischen Kohlenwasserstoffen wie Benzol oder Toluol oder offenkettigen oder cyclischen Äthern wie Dioxan, dienen. Gewünschtenfalls kann auch zunächst eine Umsetzung des Aldehydes der Formel (II) mit dem Amin der Formel (III) in einem der vorgenannten inerten aprotischen Lösungsmittel erfolgen und das erhaltene Reaktionsgemisch anschliessend mit dem nukleophilen Lösungsmittel der Formel (IV) behandelt werden.

Allfällige Benzyloxygruppen können auf an sich bekannte Weise hydrogenolytisch zu Hydroxygruppen gespalten werden, beispielsweise unter Verwendung von Palladium/Kohle als Katalysator.

Bei der cyclisierenden Umsetzung der Verbindungen der Formel (II) mit den Aminen der Formel (III) und dem Nukleophil der Formel (IV) bleibt die Konfiguration an allen Asymmetriezentren der Verbindungen der Formel (II) erhalten, so dass die Stellung der Substituenten an den Zentren $C_9$ und $C_{11}$ in den Verbindungen der Formel (I) mit der entsprechenden Konfiguration der eingesetzten Ausgangsprodukte übereinstimmt. Bei der Cyclisierung entsteht ein Gemisch von 5S- und 5R-Diastereomeren. Das Diastereomerenverhältnis kann je nach Art des verwendeten Nukleophils der Formel (IV) variieren, es wird jedoch im allgemeinen vorwiegend das 5S-Diastereomere gebildet. Bei chromatographischer Reinigung des Rohproduktes können die beiden diastereomeren Formen auf Grund ihrer unterschiedlichen Polarität aus verschiedenen Fraktionen des Eluats getrennt isoliert werden.

Die nach dem erfindungsgemässen Verfahren erhaltenen Verbindungen der Formel (I) können in an sich bekannter Weise in Form der freien Basen oder ihrer Säureadditionssalze isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel (I) eignen sich beispielsweise deren Salze mit Chlorwasserstoffsäure, Bromwasserstoffsäure, Phosphorsäure, Methansulfonsäure, Benzolsulfonsäure, p-Toluolsulfonsäure, Zitronensäure, Essigsäure, Milchsäure, Bernsteinsäure, Maleinsäure, Fumarsäure, Äpfelsäure, Weinsäure, Benzoesäure, Phenylessigsäure oder Mandelsäure.

Die Ausgangsverbindungen der Formel (II) sind Gegenstand der Parallelanmeldung EP-A 136 662. Sie können erhalten werden, indem man Verbindungen der allgemeinen Formel (V)

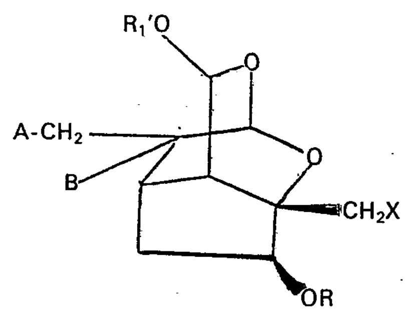

V

worin $R_1'$, A und B obige Bedeutung besitzen, X Jod oder Brom bedeutet und R Wasserstoff oder niederes Acyl bedeutet, mit einer Base in Gegenwart eines Lösungsmittels behandelt.

Vorzugsweise werden Verbindungen der Formel (V) eingesetzt, worin X Jod bedeutet und R Acetyl oder Wasserstoff bedeutet.

Verbindungen der Formel (V), worin R Wasserstoff bedeutet, können durch Hydrolyse aus Verbindungen der Formel (V), worin R Acetyl bedeutet, hergestellt werden.

Als Basen eignen sich niedere Alkalimetallalkoxide, beispielsweise Natriummethylalkoholat, Alkalimetallhydroxide oder -Carbonate, beispielsweise Kaliumcarbonat oder Alkalimetallhydride wie z.B. Natriumhydrid. Ferner sind quartäre organische Ammoniumhydroxide, beispielsweise quartäre Niederalkylammoniumhydroxide, wie Tetrabutylammoniumhydroxid, oder tertiäre organische Amine geeignet.

Zweckmässigerweise wird die Reaktion in einem Lösungsmittel, welches sowohl die Verbindung der Formel (V) wie auch die verwendete Base zu lösen vermag, durchgeführt. Als Beispiele geeigneter Lösungsmittel seien niedere Alkohole, wie z.B. Methanol oder Äthanol, offene oder cyclische Äther, wie z.B. Diäthyläther, Tetrahydrofuran oder Dioxan, oder aromatische Kohlenwasserstoffe wie Toluol oder Benzol genannt. Gegebenenfalls können diese Lösungsmittel im Gemisch mit Wasser verwendet werden. Sofern als Basen Alkalimetallalkoholate eingesetzt werden, werden als Lösungsmittel zweckmässigerweise die entsprechenden niederen Alkohole verwendet.

Die Reaktion kann bei Temperaturen zwischen 10 °C und 110 °C, vorzugsweise zwischen Raumtemperatur und 80 °C, durchgeführt werden. Die Reaktionsdauer kann je nach Art des verwendeten Ausgangsproduktes und der verwendeten Reaktionsbedingungen zwischen 1 und 5 Stunden betragen. Falls R in den Verbindungen der Formel (V) eine niedere Acylgruppe bedeutet, müssen selbstverständlich die zu verwendenden Basen und deren Mengen sowie die Reaktionsbedingungen derart gewählt werden, dass sie für eine Hydrolyse der Estergruppe geeignet sind.

Die Ausgangsverbindungen der Formel (V) und deren Herstellung sind bekannt, z.B. aus den DE-OS 2 129 507, 2 607 106 und 2 719 916, oder sie können nach den in diesen Offenlegungsschriften beschriebenen Verfahren bzw. analog zu den dort beschriebenen Verfahren hergestellt werden. Verbindungen der Formel (V), worin A und B je Wasserstoff bilden, können durch Hydrierung von Verbindungen der Formel (V), worin A und B gemeinsam eine Bindung bilden, hergestellt werden. Bei der Hydrierung wird ein Epimerengemisch erhalten, worin R- und S-Konfiguration im Verhältnis 9:1 vorliegen. Die Epimeren können durch fraktionierte Kristallisation wie in der DE-OS 2 719 916 beschrieben getrennt werden.

Die Ausgangstryptaminverbindungen der Formel (III) sind bekannt oder können nach an sich zur Herstellung substituierter Tryptamine bekannten Verfahren hergestellt werden.

Beispielsweise können die Tryptaminverbindungen der Formel (III) ausgehend von den Indolen der Formel (VI)

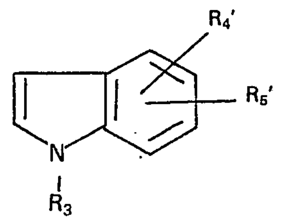

VI

worin $R_3$, $R_4'$ und $R_5'$ obige Bedeutung besitzen, erhalten werden, indem man sie zunächst in einer Mannich-Reaktion mit Formaldehyd und Dimethylamin zu entsprechenden 3-Dimethylaminomethylindolen umsetzt, diese anschliessend durch Reaktion mit Methyljodid quarternisiert, die quartären 3-Trimethylammoniummethylindolprodukte mit Alkalimetallcyanid zu den entsprechenden Indol-3-ylacetonitrilen umsetzt und diese zu den Tryptaminen der Formel (III) reduziert.

Die Mannich-Reaktion kann zweckmässigerweise mit 37%-iger Formaldehydlösung und Dimethylamin in einem Dioxan/Wasser-Gemisch unter Zusatz von Eisessig erfolgen. Für die anschliessende Quarternisierung wird vorzugsweise mit Methyljodid in Äthanol bei 0 °C umgesetzt. Als Alkalimetallcyanide eignen sich Natrium- oder Kaliumcyanide. Die Umsetzung der quartären Produkte mit dem Cyanid erfolgt vorzugsweise in einem Alkohol/Wasser-Gemisch bei erhöhter Temperatur, z.B. 80 °C. Für die Reduktion der Acetonitrile eignen sich Hydridkatalysatoren. Als günstig erweisen sich z.B. Lithiumaluminiumhydrid in Tetrahydrofuran bei 65 °C oder Diisobutylaluminiumhydrid in Methylenchlorid bei Raumtemperatur.

Indole der Formel (VI), worin $R_3$ $C_1$–$C_4$-Alkyl bedeutet, können in an sich bekannter Weise durch Alkylierung entsprechender in 1-Stellung unsubstituierter Indole mit Alkylhalogeniden oder Alkylsulfonsäureestern hergestellt werden.

Die erfindungsgemässen neuen nicht benzylsubstituierten Verbindungen der Formel (I) und ihre pharmakologisch akzeptablen Säureadditionssalze zeichnen sich durch interessante pharmakologische Eigenschaften aus. So bewirken sie insbesondere eine günstige Beeinflussung des Herzkreislaufsystems und besitzen eine hohe physiologische Verträglichkeit. Die Verbindungen zeigen insbesondere eine ausgeprägte blutdrucksenkende Wirkung, während die Herzfrequenz nur geringfügig beeinflusst wird.

Die Herzkreislauf-Wirkungen der Substanzen der Formel (I) lassen sich in pharmakologischen Standardtestmethoden an Tieren nachweisen.

Die Wirkung der Substanzen auf Blutdruck, Herzfrequenz und EKG-Parameter bei i.v.-Dauerinfusion in narkotisierten Ratten wird nach der Methode von Buschmann et al. (J. Cardiovascular Pharmacol. 2, 777–781 (1980) ) bestimmt.

Männliche Wistar-Ratten (330–370 g Körpergewicht) werden durch i.p.-Applikation von 1,25 g/kg Urethan narkotisiert und tracheotomiert. Nach einer Äquilibrierungsphase von 10 Minuten wird mit den Messungen begonnen. In einer Vorlaufphase von 5 Minuten werden die Ausgangswerte gemessen. Anschliessend werden die Prüfsubstanzen gelöst in isotonischer Natriumchloridlösung (gegebenenfalls mit Zusatz eines Lösungsvermittelers) als Dauerinfusion i.v. appliziert, beginnend mit einer Dosis von 0,01 µmol/kg/min. Die Dosis wird alle 10 Minuten ohne Erhöhung des Infusionsvolumens auf das 10-fache gesteigert. Es werden der systolische und der diastolische Blutdruck (P syst und P diast) gemessen und daraus der mittlere Blutdruck (P mittel) bestimmt, und aus dem Elektrocardiogramm (EKG) werden die Werte für die atrioventriculäre Überleitungszeit (in msec. herzfrequenz-korrigiert = PRc), das Zeitintervall der intraventriculären Erregungsausbreitung (= QRS) und die Zeitdauer vom Beginn der ventriculären Erregungsausbreitung bis zum Maximum der T-Welle (= R–αT) abgelesen. Die Herzfrequenz wird aus dem R–R Abstand bestimmt.

Aus den gemessenen Blutdruck- und Herzfrequenzwerten werden die $ED_{75}$ in µmol/kg berechnet, das ist die Gesamtdosis, welche eine Senkung dieser Parameter um 25% ihres Ausgangswertes bewirkt. Aus den dem EKG entnommenen Werten wird die jeweilige $ED_{125}$ in µmol/kg berechnet, das ist die Gesamtdosis, welche die Parameter um 25% ihres Ausgangswertes erhöht.

Ferner wird in dem Versuch auch die minimale letale Dosis (DL min) in µmol/kg mitbestimmt.

In der vorbeschriebenen Versuchsanordnung zeigen die Verbindungen der Formel (I) in einem Dosisbereich von 1 bis 100 µmol/kg blutdrucksenkende Wirkungen. So wurden z.B. für die Substanzen der nachstehenden Herstellungsbeispiele 1A, 2A und 3A die folgenden Werte ermittelt:

| Substanz Beispiel Nr. | | 1A | 2A* | 3A*∞ |
|---|---|---|---|---|
| Herzfrequenz $ED_{75}$ | µmol/kg | 41 | 21 | 100 |
| $P_{syst}$ $ED_{75}$ | µmol/kg | 21 | 6,7 | 70 |
| $P_{diast}$ $ED_{75}$ | µmol/kg | 5,9 | 3,9 | 30–35 |
| $P_{mittel}$ $ED_{75}$ | µmol/kg | 9,7 | 4,5 | 40 |
| $PR_c$ $ED_{125}$ | µmol/kg | 93 | 42 | 40 |
| QRS $ED_{125}$ | µmol/kg | 35 | 28 | 45 |
| R–αT $ED_{125}$ | µmol/kg | 19 | 6,7 | 20 |
| $DL_{min}$ | µmol/kg | 210 | 100 | >210 |

* Lösungsvermittlerzusatz

∞ Lösungsvermittler zeigt in oberen Dosen schwache blutdrucksteigernde Eigenwirkung.

Aus diesen Werten ist ersichtlich, dass sich das Wirkungsprofil der erfindungsgemässen Substanzen durch eine hohe Spezifität der blutdrucksenkenden Wirkung insbesondere auf den diastolischen Blutdruck und eine hohe Verträglichkeit auszeichnet.

Aufgrund ihrer spezifisch blutdrucksenkenden Eigenschaften sind die Substanzen als Antihypertensiva zur Behandlung von Bluthochdruck geeignet.

Als Heilmittel können die nicht benzylsubstituierten Verbindungen der Formel (I) und ihre physiologisch verträglichen Säureadditionssalze zusammen mit üblichen pharmazeutischen Träger- und/oder Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe, wie z.B. Milchzucker, Stärke oder Talkum oder flüssiger Verdünnungsmittel, wie z.B. Wasser, fette Öle oder flüssige Paraffine.

Die nachfolgenden Beispiele sollen die Erfindung näher erläutern.

Beispiel A

Herstellung des Ausgangsproduktes:

1R, 4S, 5R, 7R-5-Methoxy-3,8-dimethylen-2,6-dioxa-bicyclo-[2,2,2]-octan-7-yl-acetaldehyd.

a) Eine Lösung von 7,6 g 1R, 3S, 4S, 6R, 7S, 8R-3- Jodmethyl- 4-acetoxy- 8-methoxy-10-methylen-2,9- dioxa- tricyclo-$[4,3,1,0^{3,7}]$-decan in 100 ml absolutem Methanol wird zu einer durch Auflösen von 0,44 g Natrium in 40 ml absolutem Methanol bereiteten Natriummethylat-Lösung gegeben. Das Gemisch wird 4,5 Stunden lang bei 60 °C reagieren gelassen. Anschliessend wird zur Aufarbeitung auf Eiswasser gegossen, mit Natriumchlorid gut ausgesalzen und mit Äther extrahiert. Die Ätherphase wird mit einigen Tropfen Eisessig versetzt, über Natriumsulfat getrocknet, filtriert und eingeengt. Als Rückstand verbleiben 4,29 g rohes 1R, 4S, 5R, 7R-5-Methoxy-3,8-dimethylen-2,6-dioxa- bicyclo-[2,2,2]-octan-7-yl-acetaldehyd.

Das Rohprodukt kann bereits in dieser Form als Zwischenprodukt zur weiteren Umsetzung zu einer Verbindung der Formel (I) verwendet werden.

Zur Reindarstellung wird das Rohprodukt chromatographisch über Kieselgel unter Verwendung von n-Hexan/Äther als Elutionsmittel gereinigt. Das nach Einengen des Eluates erhaltene dünnschichtchromatographisch reine Produkt wird aus n-Hexan/Äther kristallisiert.

Fp. 56–59 °C

IR-Spektrum: 3070 $cm^{-1}$, 1725 $cm^{-1}$, 1675 $cm^{-1}$, 1170 $cm^{-1}$, 1075 $cm^{-1}$, 960 $cm^{-1}$.

b) 6 g 1R, 3S, 4S, 6R, 7S, 8R-3-Jodmethyl-4-acetoxy-8-methoxy-10-methylen-2,9- dioxa-tricyclo-[4,3,1,$0^{3,7}$]-decan werden zu einer durch Auflösen von 2,5 g Natrium in 250 ml absolutem Methanol bereiteten Natriummethylat-Lösung gegeben und das Reaktionsgemisch 1,5 Stunden bei einer Badtemperatur von 60 °C unter Stickstoff gerührt. Zur Aufarbeitung wird anschliessend auf ca. 1/4 des ursprünglichen Volumens am Rotationsverdampfer eingeengt und die eingeengte Lösung vorsichtig mit 100 g gesättigter $(NH_4)_2SO_4$-Lösung versetzt, wobei $NH_3$ entweicht. Anschliessend wird fünfmal mit insgesamt 500 ml Äther extrahiert. Die ätherischen Extrakte werden mit 50 ml $(NH_4)_2SO_4$-Lösung und anschliessend mit Wasser gewaschen, über Magnesiumsulfat getrocknet und eingedampft. Es werden 2,9 g rohes 1R, 4S, 5R, 7R-5-Methoxy-3,8-dimethylen- 2,6-dioxa- bicyclo- [2,2,2]-octan-7-yl-acetaldehyd erhalten.

Dieses Rohprodukt kann ohne weitere Reinigung als Zwischenprodukt zur Herstellung von Verbindungen der Formel (I) verwendet werden. Gewünschtenfalls kann das Produkt, wie in Beispiel Aa beschrieben, weiter gereinigt werden. Das gereinigte Produkt stimmt mit dem in Beispiel Aa erhaltenen Produkt überein.

Beispiel B

Herstellung des Ausgangsproduktes:

1R, 4S, 5R, 7R, 8R-5-Methoxy-8-methyl-3-methylen-2,6-dioxabicyclo-[2,2,2]-octan-7-yl-acetaldehyd.

a) Eine Lösung von 10 g 1R, 3S, 4S, 6R, 7S, 8R, 10R-3-Jodmethyl-4- acetoxy-8- methoxy-10-methyl-2,9-dioxa-tricyclo-[4,3,1,$0^{3,7}$]-decan in 220 ml Methanol wird mit 3,6 g Kaliumcarbonat versetzt und die Reaktionsmischung ca. 1 Stunde bei Raumtemperatur gerührt. Zur Aufarbeitung wird das Gemisch mit Wasser verdünnt und mit Methylenchlorid extrahiert. Die organische Phase wird abgetrennt, über Natriumsulfat getrocknet, filtriert und im Vakuum zur Trockne eingeengt. Es werden 8,7 g 1R, 3S, 4S, 6R, 7S, 8R, 10R- 3-Jodmethyl-4- hydroxy-8- methoxy-10- methyl-2,9-dioxatricyclo-[4,3,1,$0^{3,7}$]-decan erhalten. Fp.: 92–93 °C.

b) Eine Lösung von 3,24 g der vorstehenden Verbindung in 50 ml absolutem Methanol werden zu einer durch Auflösen von 0,23 g Natrium in 50 ml absolutem Methanol gebildeten Natriummethylat-Lösung gegeben. Das Reaktionsgemisch wird ca. 3 Stunden bei einer Temperatur von 60 °C gerührt. Anschliessend wird zur Aufarbeitung auf Eiswasser gegossen, gut mit Natriumchlorid ausgesalzen und mit Äther extrahiert. Die Ätherphase wird mit einigen Tropfen Eisessig versetzt, über Natriumsulfat getrocknet, filtriert und im Vakuum bis zur Trockne eingeengt. Als Rückstand werden 2,7 g rohes 1R, 4S, 5R, 7R, 8R-5-Methoxy-8- methyl-3- methylen-2,6-dioxa-bicyclo-[2,2,2]-octan-7- yl- acetaldehyd erhalten.

Dieses Rohprodukt kann ohne weitere Reinigung zur Herstellung von Verbindungen der Formel (I) verwendet werden.

Beispiel C

Herstellung des Ausgangsproduktes:

1R, 4S, 5R, 7R-5-Benzyloxy-3,8-dimethylen-2,6-dioxabicyclo- [2,2,2]-octan -7-yl-acetaldehyd.

8,5 g 1R, 3S, 4S, 6R, 7S, 8R-3-Jodmethyl-4-acetoxy-8- benzyloxy-10-methylen-2,9-dioxatricyclo[4,3,1,$0^{3,7}$]-decan (Fp.: 69–70 °C, hergestellt auf an sich bekannte Weise durch Umsetzung von Didrovaltratum mit Benzylalkohol und Jodwasserstoffsäure) werden mit einer durch Auflösen von 2,5 g Natrium in 300 ml absolutem Methanol bereiteten Natriummethylatlösung, wie in Beispiel A Beschrieben, umgesetzt. Zur Aufarbeitung wird das Reaktionsgemisch auf Eiswasser gegossen, mit Methylenchlorid extrahiert und die organische Phase abgetrennt und wie in Beispiel A beschrieben aufgearbeitet. Als Rückstand werden 6 g rohes 1R, 4S, 5R, 7R-5-Benzyloxy-3,8-dimethylen- 2,6-dioxa-bicyclo-[2,2,2]-octan-7-yl-acetaldehyd erhalten.

Dieses Rohprodukt kann ohne weitere Reinigung zur Herstellung von Verbindungen der Formel (I) verwendet werden.

Beispiel 1

Herstellung von 1R, 3R, 5S, 7R, 8R, 9R- und 1R, 3R, 5R, 7R, 8R, 9R-N-(3,9-Dimethoxy-11-methylen- 2,10-dioxa- tricyclo- [5,3,1,$0^{3,8}$]-undecan-5-yl)-tryptamin.

A. Aus 4 g 1R, 3S, 4S, 6R, 7R, 8R-3-Jodmethyl- 4-acetoxy- 8-methoxy- 10-methylen-2,9-dioxa-tricyclo-[4,3,1,$0^{3,7}$]-decan nach der Vorschrift des Beispiels Ab erhaltenes rohes 1R, 4S, 5R, 7R-5-Methoxy-3,8-dimethylen-2,6-dioxa-bicyclo-[2,2,2]-octan-7-yl-acetaldehyd werden in 100 ml absolutem Methanol gelöst und die Lösung unter Stickstoff mit 2 g Tryptaminhydrochlorid versetzt und das Reaktionsgemisch bei einer Badtemperatur von 60 °C 3 1/2 Stunden gerührt. Anschliessend wird zur Aufarbeitung das Methanol abdestilliert, das verbleibende Reaktionsgemisch mit konzentrierter Sodalösung alkalisiert und mit Methylenchlorid extrahiert. Die organische Phase wird über Magnesiumsulfat getrocknet und eingedampft. Es werden 4 g rötlich gefärbtes Rohprodukt erhalten. Dieses wird durch Säulenchromatographie unter Druck unter Verwendung von Chloroform enthaltend 5% Methanol als Elutionsmittel gereinigt. Aus dem Eluat werden 890 mg unpolares 1R, 3R, 5S, 7R, 8R, 9R-N- (3,9- Dimethoxy- 11-methylen- 2,10-dioxa-tricyclo-[5,3,1,$0^{3,8}$]-undecan-5-yl)-tryptamin als farbloses Schaumharz erhalten.

$[\alpha]_D^{20} = +3{,}3°$ (C = 1 in $CH_3OH$)

Molekulargewicht:
berechnet 384,2049 gefunden 384,2048
Massenspektrum (130 °C):
$M^+$ = 384(4), 383(11), 352(11), 253(43), 221(21), 192(27), 179(42), 175(9), 144(11), 131(100).

B. Ferner wird aus polareren Fraktionen des Eluats als polares Nebenprodukt rohes 1R, 3R, 5R, 7R, 8R, 9R-N-(3,9-Dimethoxy-11-methylen-2,10-dioxa-tricyclo-[5,3,1,$0^{3,8}$]-undecan-5-yl)-tryptamin erhalten. Nach nochmaliger Reinigung durch präparative Dünnschichtchromatographie unter Verwendung von Chloroform enthaltend 5% Methanol als Elutionsmittel werden 125 mg reines Produkt als farbloses Schaumharz erhalten.

Molekulargewicht:
berechnet 384,2049 gefunden 384,2048
Massenspektrum (110 °C):
$M^+$ = 384(7), 353(8), 254(100), 242(12), 222(27), 196(28), 166(38), 144(28), 130(52)

Beispiel 2

Herstellung von 1R, 3R, 5S, 7R, 8R, 9R-N-(3-tert.-Butoxy-9-methoxy-11-methylen-2,10-dioxa-tricyclo-[5,3,1,$0^{3,8}$]-undecan-5-yl)-tryptamin und 1R, 3R, 5R, 7R, 8R, 9R-N-(3-tert.-Butoxy-9-methoxy-11-methylen-2,10-dioxa-tricyclo-[5,3,1,$0^{3,8}$]-undecan-5-yl)-tryptamin.

A. 580 mg rohes 1R, 4S, 5R, 7R-5-Methoxy-2,8-dimethylen- 2,6-dioxa- bicyclo- [2,2,2]-octan-7-yl-acetaldehyd (hergestellt analog Beispiel A.b) werden in einer Mischung aus 10 ml absolutem Dioxan und 30 ml absolutem tert.-Butanol gelöst und die Lösung mit 540 mg Tryptaminhydrochlorid versetzt. Das Reaktionsgemisch wird bei einer Badtemperatur von 60 bis 70 °C 4 Stunden gerührt. Anschliessend wird zur Aufarbeitung das Lösungsmittel abdestilliert, das verbleibende Reaktionsgemisch mit konzentrierter Sodalösung alkalisiert und mit Chloroform extrahiert. Die organische Phase wird über Magnesiumsulfat getrocknet und eingedampft. Es werden 1,1 g Rohprodukt erhalten. Dieses wird durch Säulenchromatographie und Druck unter Verwendung von Chloroform enthaltend 5% Methanol als Elutionsmittel gereinigt. Aus dem Eluat wurden 270 mg unpolares reines 1R, 3R, 5S, 7R, 8R, 9R-N-(3-tert.-Butoxy-9-methoxy-11-methylen-2,10-dioxa-tricyclo[5,3,1,$0^{3,8}$]-undecan-5-yl)-tryptamin als Schaumharz erhalten.

$[\alpha]_D^{20} = -8°$ (C = 1 in $CH_3OH$)

Massenspektrum (160 °C):
$M^+$ = (426(1), 425(3), 395(1), 353(3), 339(4), 296(4), 222(57), 193(20), 180(73), 165(12), 144(19), 131(100).

B. Ferner wird aus polareren Fraktionen des Eluats als polares Nebenprodukt rohes 1R, 3R, 5R, 7R, 8R, 9R-N-(3-Butoxy-9-methoxy-11-methylen-2,10-dioxa-tricyclo-[5,3,1,$0^{3,8}$]-undecan-5-yl)-tryptamin erhalten. Nach nochmaliger Reinigung durch präparative Dünnschichtchromatographie unter Verwendung von Chloroform enthaltend 10% Methanol als Elutionsmittel werden 34 mg reines Produkt erhalten.

Massenspektrum (80 °C):
$M^+$ = 426(3), 411(8), 406(6), 393(3), 333(100), 319(10), 302(21), 288(28), 226(19), 222(391).

Beispiel 3

Herstellung von 1R, 3R, 5S, 7R, 8R, 9R-N-(3-Acetoxy- 9-methoxy- 11-methylen- 2,10-dioxa-tricyclo-[5,3,1,$0^{3,8}$] -undecan-5-yl)-tryptamin und 1R, 3R, 5R, 7R, 8R, 9R-N-(3-Acetoxy-9-methoxy-11-methylen-2,10-dioxa-tricyclo-[5,3,1,$0^{3,8}$]-undecan-5-yl)-tryptamin.

A. 2,5 g Tryptamin werden in 50 ml einer Mischung aus absolutem Toluol und Dioxan im Verhältnis 4 zu 1 bei 100 °C Badtemperatur gelöst und zum Abkühlen stehengelassen. Zu der noch warmen Lösung wird eine Lösung von 2,5 g rohem 1R, 4S, 5R, 7R-5-Methoxy-3,8-dimethylen-2,6-dioxa-bicyclo-[2,2,2]-octan- 7-yl-acetaldehyd (hergestellt nach Beispiel A.b) in 20 ml Toluol gegeben. Es entsteht eine Trübung. Es werden 1 g Magnesiumsulfat zugesetzt und das Reaktionsgemisch wird weitere 24 Stunden bei Raumtemperatur gerührt. Anschliessend wird filtriert, mit Methylenchlorid nachgespült, eingedampft und der Rückstand in 50 ml trockenem frisch destilliertem Eisessig 12 Stunden bei Raumtemperatur unter Stickstoffatmosphäre stehengelassen. Zur Aufarbeitung wird nach vorsichtigem Abdampfen des Lösungsmittels auf dem Wasserbad das verbleibende Reaktionsgemisch mit konzentrierter Sodalösung alkalisiert und sofort mit Methylenchlorid extrahiert. Die organische Phase wird mit Wasser gewaschen, über Magnesiumsulfat getrocknet und eingedampft. Es werden 4,9 g Rohprodukt erhalten. Dieses wird durch Säulenchromatographie unter Druck unter Verwendung von Chloroform enthaltend 5 bis 10% Methanol als Elutionsmittel gereinigt. Aus dem Eluat werden 710 mg unpolares 1R, 3R, 5S, 7R, 8R, 9R-N-(3-Acetoxy-9-methoxy-11-methylen-2,10-dioxa-tricyclo-[5,3,1,$0^{3,8}$]-undecan-5-yl)-tryptamin als farbloses Schaumharz erhalten.

$[\alpha]_D^{20} = +1{,}1°$ (C = 1 in $CH_3OH$)

Molekulargewicht:
berechnet 412,1998 gefunden 412,1992
Massenspektrum (130 °C):
$M^+$ = 412(5), 381(3), 353(4), 321(4),

228(13), 222(36), 213(9), 210(8), 193(12), 180(43), 171(19), 165(10), 156(6), 148(11), 144(41), 131(100).

B. Aus den polareren Fraktionen des Eluats wird als polares Nebenprodukt rohes 1R, 3R, 5R, 7R, 8R, 9R-N-(3-Acetoxy-9-methoxy-11-methylen-2,10-dioxa-tricyclo-[5,3,1,$0^{3,8}$]-undecan-5-yl)-tryptamin erhalten. Nach nochmaliger Reinigung durch präparative Dünnschichtchromatographie unter Verwendung von Chloroform enthaltend Methanol als Elutionsmittel werden 105 mg reines Produkt erhalten.

Molekulargewicht:
berechnet 412,1998 gefunden 412,1996
Massenspektrum (160 °C):
$M^+$ = 412(4), 381(2), 352(3), 321(3), 228(7), 222(42), 213(5), 210(8), 193(15), 180(40), 171(11), 165(9), 148(12), 144(37), 131(100).

Beispiel 4

Herstellung von 1R, 3R, 5S, 7R, 8R, 9R- und 1R, 3R, 5R, 7R, 8R, 9R-N-(3,9-dimethoxy-11-methyl-2,10-dioxa-tricyclo-[5,3,1,$0^{3,8}$]-undecan-5-yl)-5'-methoxytryptamin.

2,7 g 1R, 4S, 5R, 7R, 8R-5-Methoxy-8-methyl-3-methylen-2,6-dioxa-bicyclo-[2,2,2]-octan-7-yl-acetaldehyd (hergestellt nach Beispiel B) werden in Methanol gelöst und mit 1 g 5-Methoxytryptamin nach dem in Beispiel 1A beschriebenen Verfahren umgesetzt. Es werden 1,3 g Rohprodukt erhalten. Dieses wird wie in Beispiel 1 beschrieben durch Säulenchromatographie gereinigt. Das gereinigte Rohprodukt wird durch präparative Dünnschichtchromatographie weiter gereinigt unter Verwendung von Methylenchlorid enthaltend 20% Methanol als Elutionsmittel. Hierbei werden 200 mg reines 1R, 3R, 5S, 7R, 8R, 9R-N-(3,9-Dimethoxy-11-methyl-2,10-dioxatricyclo-[5,3,1,$0^{3,8}$]-undecan-5-yl)-5-methoxytryptamin als farbloses Schaumharz erhalten.

$$[\alpha]_D^{20} = -14{,}2° \ (C = 1 \text{ in } CH_3OH)$$

Massenspektrum (190 °C):
$M^+$ = 416(3), 385(5), 256(15), 224(40), 216(12), 182(13), 174(29), 173(26), 168(16), 162(22), 161(100), 160(44), 161(100), 160(44).

Die restliche Substanz wird als Schaumharz erhalten, welches ein Epimerengemisch aus dem 5S- und dem 5R-Epimeren darstellt.

Beispiel 5

Herstellung von 1R, 3R, 5S, 7R, 8R, 9R-N-(3,9-Dimethoxy-11-methyl-2,10-dioxa-tricyclo-[5,3,1,$0^{3,8}$]-undecan-5-yl)-5-benzyloxytryptamin.

1,2 g 1R, 4S, 5R, 7R, 8R-5-Methoxy-8-methyl-3-methylen-2,6-dioxa-bicyclo-[2,2,2]-octan-7-yl-acetaldehyd werden in Methanol gelöst mit 1,0 g 5-Benzyloxytryptamin wie in Beispiel 1A beschrieben umgesetzt. Das erhaltene Rohprodukt wird durch Säulenchromatographie wie in Beispiel 1 beschrieben gereinigt. Es werden 1,5 g reines 1R, 3R, 5S, 7R, 8R, 9R-N-(3,9-Dimethoxy-methyl-12,10-dioxa-tricyclo-[5,3,1,$0^{3,8}$]-undecan-5-yl)-5-benzyloxytryptamin als farbloses Schaumharz erhalten.

$$[\alpha]_D^{20} = -30{,}2° \ (C = 1 \text{ in } CH_3OH)$$

Massenspektrum (190 °C)
$M^+$ = 492(3), 461(6), 292(15), 256(17), 238(34), 237(100), 236(18), 224(43), 167(13), 146(56), 145(13), 91(29).

Beispiel 6

Herstellung von 1R, 3R, 5S, 7R, 8R, 9R- und 1R, 3R, 5R, 7R, 8R, 9R-N-(3-methoxy-9-benzyloxy-11-methylen-2,10-dioxa-tricyclo-[5,3,1,$0^{3,8}$]-undecan-5-yl)-tryptamin.

6 g 1R, 4S, 5R, 7R-5-Benzyloxy-3,8-dimethylen-2,6-dioxa-bicyclo-[2,2,2]-octan-7-yl-acetaldehyd (hergestellt nach Beispiel C) werden in 100 ml Methanol gelöst und die Lösung portionsweise mit 3,6 g Tryptaminhydrochlorid versetzt und das Reaktionsgemisch bei einer Badtemperatur von 60 °C 2 Stunden gerührt. Anschliessend wird zur Aufarbeitung im Vakuum zur Trockne eingeengt. Als Rückstand verbleibt das rohe Hydrochlorid des Epimerengemisches der Titelverbindungen. Dieses wird säulenchromatographisch über Kieselgel aufgetrennt. Als Elutionsmittel wird n-Hexan mit steigenden Mengen (bis zu 100%) Methylenchlorid und anschliessend Methylenchlorid mit bis zu 5% ansteigenden Mengen Methanol verwendet. In einer ersten Eluatfraktion werden 0,57 g nur wenig verunreinigtes 1R, 3R, 5S, 7R, 8R, 9R-N-(3-Methoxy-9-benzyloxy-11-methylen-2,10-dioxa-tricyclo-[5,3,1,$0^{3,8}$]-undecan-5-yl)-tryptamin erhalten. Dieses wird nochmals über Kieselgel unter Verwendung von Methylenchlorid enthaltend bis zu 5% Methanol als Elutionsmittel gereinigt. Es werden 0,33 g reines 5S-Epimeres als Schaumharz erhalten.

$$[\alpha]_D^{20} = -22{,}1° \ (C = 1 \text{ in } CH_3OH)$$

Zur Überführung in die freie Base wird das Hydrochlorid in Dichlormethan gelöst und mit einem Äquivalent Bariumhydroxid ($Ba(OH)_2 \cdot 8H_2O$) versetzt. Das Reaktionsgemisch wird 30 Minuten bei Raumtemperatur gerührt, mit Natriumsulfat getrocknet und filtriert. Anschliessend wird im Vakuum zur Trockne eingeengt. Die freie Base wird als Schaumharz erhalten.

$$[\alpha]_D^{20} = 18{,}3 \ (c = 1 \text{ in } CH_3OH)$$

Massenspektrum (190 °C)
$M^+$ = 460(0,6), 423(0,6), 330(16), 298(26), 144(14), 132(14), 131(95), 130(28), 95(7), 92(10), 91(100), 77(6).

Ferner werden aus einer zweiten polareren Fraktion des obigen Eluats 1,7 g eines rohen Epimerengemisch-Hydrochlorides enthaltend ca. 80% 5S-Epimeres und ca. 20% 5R-Epimeres und aus einer dritten noch polareren Fraktion des Eluates 2,8 g eines rohen Epimerengemisch-Hydrochlorides enthaltend das 5S- und das 5R-Epimere zu etwa gleichen Teilen erhalten. Diese Epimerengemische können gewünschtenfalls durch präparative Dünnschichtchromatographie in die beiden Epimeren aufgetrennt werden.

Beispiel 7

Herstellung von 1R, 3R, 5S, 7R, 8R, 9R- und 1R, 3R, 5R, 7R, 8R, 9R-N-(3-Methoxy-9-isobutyloxy- 11-methylen- 2,10-dioxa-tricyclo-[5,3,1,$0^{3,8}$]-undecan-5-yl)-tryptamin.

11 g rohes 1R, 4S, 5R, 7R-5-Isobutyloxy-3,8-dimethylen- 2,6-dioxa-bicyclo-[2,2,2]-octan-7-yl-acetaldehyd (hergestellt analog Beispiel C aus durch Umsetzung von Didrovaltratum mit Isobutylalkohol und Jodwasserstoffsäure erhaltenem 1R, 3S, 4S, 6R, 7S, 8R-3-Jodmethyl-4-acetoxy-8-isobutyloxy- 10-methylen-2,9-dioxa-tricyclo-[4,3,1,$0^{3,7}$]-decan, Fp.: 45–50 °C, durch Ringspaltung) werden in 200 ml Methanol gelöst und die Lösung portionsweise mit 8 g Tryptaminhydrochlorid versetzt und das Reaktionsgemisch bei einer Badtemperatur von 60 °C drei Stunden gerührt. Anschliessend wird wie in Beispiel 6 beschrieben aufgearbeitet. In einer ersten Eluatfraktion werden 3,2 g nur wenig 5R-Epimeres enthaltendes 1R, 3R, 5S, 7R, 8R, 9R-N-(3-Methoxy--9-isobutyloxy -11-methylen -2,10-dioxa- tricyclo-[5,3,1,$0^{3,8}$] -undecan-5-yl)-tryptaminhydrochlorid erhalten. Dieses wird nochmals über Kieselgel unter Verwendung von Methylenchlorid enthaltend bis zu 4% Methanol als Elutionsmittel gereinigt. Es werden 1,3 g gereinigtes Produkt erhalten. Nach Umkristallisation aus Methylenchlorid/n-Hexan werden 0,6 g reines 5S-epimeres Hydrochlorid erhalten, Fp.: 205–209 °C.

$[\alpha]_D^{20} = -16,80$ °c = 1 in $CH_3OH$)

Aus dem Hydrochlorid wird wie in Beispiel 6 beschrieben die freie Base als Schaumharz erhalten.

$[\alpha]_D^{20} = -13,0°$ (c = 1 in $CH_3OH$)

Ferner werden aus einer zweiten polareren Fraktion des obigen Eluates 6,5 g eines rohen Epimerengemisch-Hydrochlorides erhalten, in welchem das 5S- und das 5R-Epimere zu etwa gleichen Teilen vorliegen.

Beispiel 8

Herstellung von 1R, 3R, 5S, 7R, 8R, 9R-N-(3-methoxy- 9-hydroxy- 11-methylen- 2,10-dioxa-tricyclo-[5,3,1,$0^{3,8}$]-undecan-5-yl)-tryptamin.

0,6 g 1R, 3R, 5S, 7R, 8R, 9R-N-(3-methoxy-9-benzyloxy- 11-methylen- 2,10-dioxa-tricyclo-[5,3,1,$0^{3,8}$]-undecan-5-yl)-tryptamin werden in 15 ml Methanol gelöst und mit einer Lösung von 0,3 ml konzentrierter Salzsäure in 5 ml Methanol versetzt. Dann werden 0,215 g eines Palladiumkatalysators (Palladium auf Kohle 5%-ig) hinzugegeben und die Mischung 2 Stunden bei Raumtemperatur unter einem Wasserstoffdruck von 3 bar hydriert. Nach Beendigung der Wasserstoffaufnahme wird der Katalysator abfiltriert und die filtrierte Lösung im Vakuum zur Trockne eingedampft. Es werden 0,58 g dünnschichtchromatografisch reines 1R, 3R, 5S, 7R, 8R, 9R-N-(3-methoxy- 9-hydroxy- 11-methylen- 2,10-dioxa-tricyclo[5,3,1,$0^{3,8}$]-undecan-5-yl)-tryptamin als Schaumharz erhalten.

$[\alpha]_D^{24} = -23$ ° (c = 1 in $CH_3OH$)

Beispiel I: Tabletten

Man stellt Tabletten in folgender Zusammensetzung pro Tablette her:

| | |
|---|---|
| 1R, 3R, 5S, 7R, 8R, 9R-N-(3,9-Dimethoxy-11-methylen-2,10-dioxa-tricyclo-[5,3,1,$0^{3,8}$]-undecan-5-yl)-tryptamin | 10 mg |
| mikrokristalline Cellulose (Avicel[R] PH101) | 93 mg |
| hochdisperse Kieselsäure (Aerosil[R] R972) | 12,5 mg |
| Carboxymethylcellulose (Tylose[R]) | 7 mg |

Der Wirkstoff wird in Methylenchlorid gelöst. Die mikrokristalline Cellulose und die hochdisperse Kieselsäure werden gemischt und mit der Wirkstofflösung verrieben. Die erhaltene Verreibung wird getrocknet und mit einer wässrigen Tylose-Lösung befeuchtet. Das erhaltene feuchte Granulat wird durch eine Sieb mit 2 mm Maschenweite gepresst, im Wirbelschichttrockner bei 40–45 °C getrocknet und nochmals durch ein Sieb mit 1,5 mm Maschenweite gegeben und anschliessend in einem Mixer mit folgenden weiteren Hilfsstoffen vermischt:

| | |
|---|---|
| Quervernetztes Polyvinylpyrrolidon (Crospovidone USP20/NF153) | 4,5 mg |
| Magnesiumstearat | 1,5 mg |
| hochdisperse Kieselsäure | 1,5 mg |

und sodann zu Tabletten zu 130 mg verpresst.

**Patentansprüche**

1.Verbindungen der allgemeinen Formel (I)

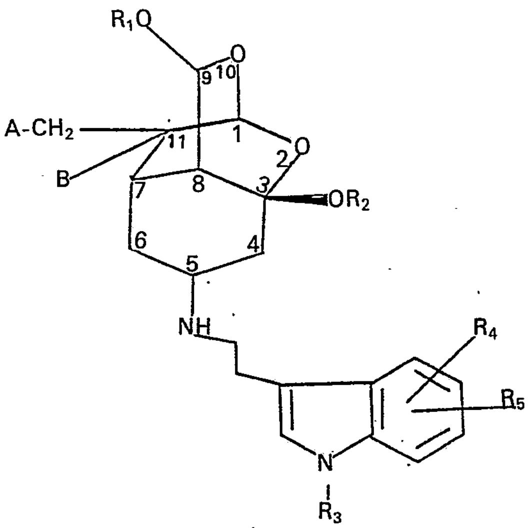

I

worin $R_1$ Wasserstoff, Alkyl mit 1–4 Kohlenstoffatomen oder Benzyl bedeutet,

$R_2$ Alkyl mit 1–4 Kohlenstoffatomen oder Alkanoyl mit 1–4 Kohlenstoffatomen bedeutet,

$R_3$ Wasserstoff oder Alkyl mit 1–4 Kohlenstoffatomen bedeutet,

$R_4$ Wasserstoff, Alkoxy mit 1–4 Kohlenstoffatomen, Benzyloxy oder Hydroxy bedeutet,

$R_5$ Wasserstoff, Alkoxy mit 1–4 Kohlenstoffatomen, Benzyloxy oder Hydroxy bedeutet, und

A und B je Wasserstoff bedeuten oder gemeinsam eine Bindung bilden, und deren Säureadditionssalze.

2. Verbindungen der Formel (I) gemäss Anspruch 1, worin $R_1$ Alkyl mit 1–4 Kohlenstoffatomen bedeutet.

3. Verbindungen der Formel (I) gemäss Anspruch 2, worin $R_3$ Wasserstoff bedeutet.

4. Verbindungen der Formel (I) gemäss Anspruch 3, worin $R_1$ und $R_2$ Alkyl mit 1–4 Kohlenstoffatomen und $R_3$, $R_4$ und $R_5$ Wasserstoff bedeuten.

5. Verbindungen der Formel (I) gemäss Anspruch 1, 2, 3 oder 4, worin A und B gemeinsam eine Bindung bilden.

6. Verfahren zur Herstellung von Verbindungen der allgemeinen Formel (I)

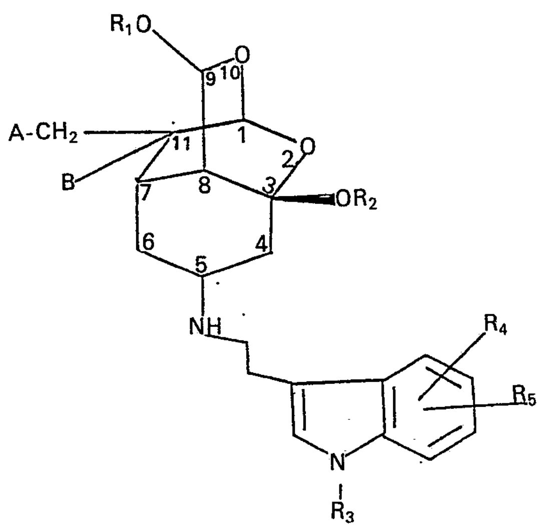

I

worin $R_1$, $R_2$, $R_3$, $R_4$, $R_5$, A und B die im Anspruch 1 angegebene Bedeutung besitzen, und deren Säureadditionssalzen, dadurch gekennzeichnet, dass man Verbindungen der allgemeinen Formel (II)

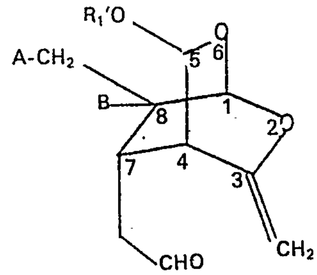

II

worin A und B obige Bedeutung besitzen und $R_1'$ niederes Alkyl oder Benzyl bedeutet, mit einem Amin der allgemeinen Formel (III)

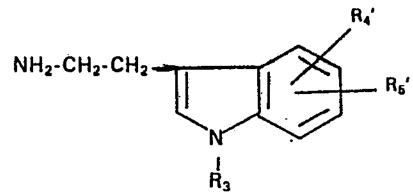

III

worin $R_3$ obige Bedeutung besitzt und $R_4'$ und $R_5'$ die für $R_4$ und $R_5$ genannten Bedeutungen mit Ausnahme von Hydroxy besitzen, und einem nukleophilen Lösungsmittel der allgemeinen Formel (IV)

$R_2OH$ IV

worin $R_2$ obige Bedeutung besitzt, umsetzt, und in den erhaltenen Verbindungen der allgemeinen Formel (Ia)

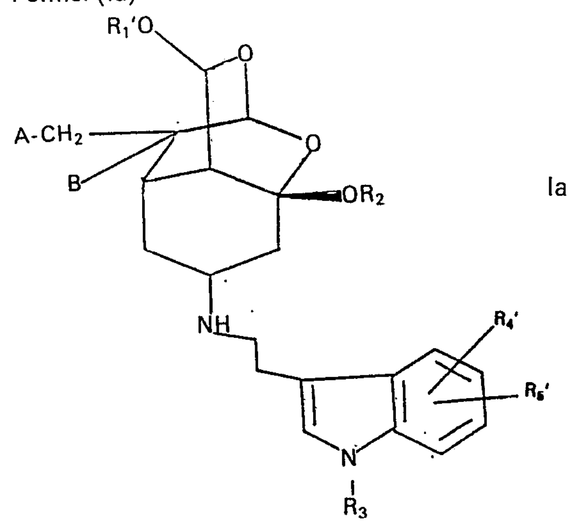

Ia

worin A, B, $R_1'$, $R_2$, $R_3$, $R_4'$ und $R_5'$ obige Bedeutung besitzen, gewünschtenfalls allfällige Benzyloxygruppen hydrogenolytisch zu Hydroxygruppen spaltet und/oder gegebenenfalls freie Verbindungen der Formel (I) in ihre Säureadditionssalze überführt oder die Säureadditionssalze in die freien Verbindungen der Formel (I) überführt.

7. Arzneimittel enthaltend eine pharmakologisch wirksame Menge einer Verbindung der Formel (I) gemäss Anspruch 1, worin $R_1$ die in Anspruch 1 angegebene Bedeutung mit Ausnahme von Benzyl, $R_4$ und $R_5$ die in Anspruch 1 angegebene Bedeutung mit Ausnahme von Benzyloxy und $R_2$, $R_3$, A und B die in Anspruch 1 angegebene Bedeutung besitzen, und übliche Hilfs- und/oder Trägerstoffe.

**Revendications**

1. Composés de formule générale (I)

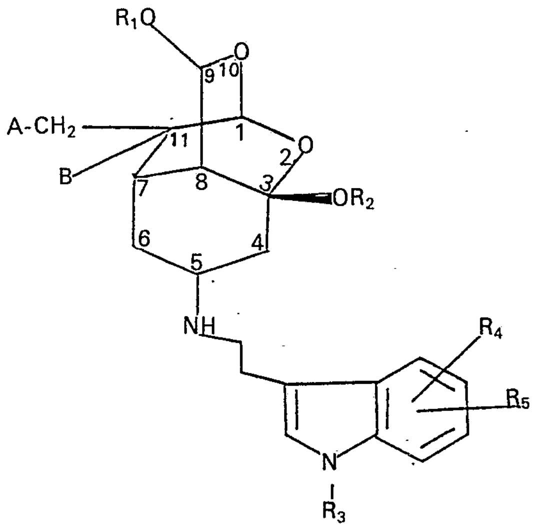

I

dans laquelle $R_1$ représente un atome d'hydrogène, un groupe alkyle ayant 1 à 4 atomes de carbone ou un groupe benzyle,

$R_2$ représente un groupe alkyle ayant 1 à 4 atomes de carbone ou alcanoyle ayant 1 à 4 atomes de carbone,

$R_3$ représente un atome d'hydrogène ou un groupe alkyle ayant 1 à 4 atomes de carbone,

$R_4$ représente un atome d'hydrogène, un groupe alcoxy ayant 1 à 4 atomes de carbone, benzyloxy ou hydroxyle,

$R_5$ représente un atome d'hydrogène, un groupe alcoxy ayant 1 à 4 atomes de carbone, benzyloxy ou hydroxyle, et

A et B représentent chacun un atome d'hydrogène ou forment ensemble une liaison, et leurs sels d'addition d'acides.

2. Composés de formule (I) selon la revendication 1, dans lesquels $R_1$ représente un groupe alkyle ayant 1 à 4 atomes de carbone.

3. Composés de formule (I) selon la revendication 2, dans lesquels $R_3$ représente un atome d'hydrogène.

4. Composés de formule (I) selon la revendication 3, dans lesquels $R_1$ et $R_2$ représentent des groupes alkyles ayant 1 à 4 atomes de carbone, et $R_3$, $R_4$ et $R_5$ représentent des atomes d'hydrogène.

5. Composés de formule (I) selon la revendication 1, 2, 3 ou 4, dans lesquels A et B forment ensemble une liaison.

6. Procédé de préparation des composés de formule générale (I)

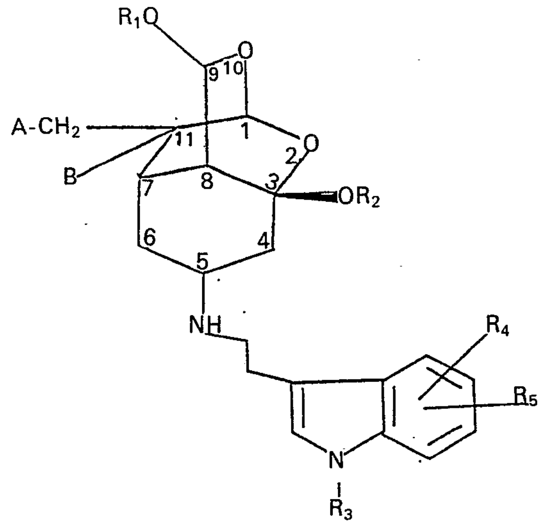

I

(dans laquelle $R_1$, $R_2$, $R_3$, $R_4$, $R_5$, A et B ont le sens indiqué à la revendication 1) et leurs sels d'addition d'acides, caractérisé en ce qu'on fait réagir des composés de formule générale (II)

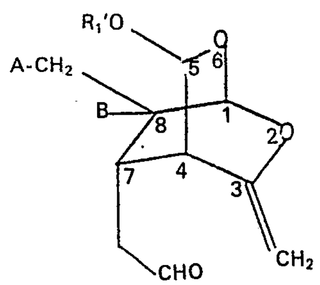

II

(dans laquelle A et B ont le sens précité et $R_1'$ représente un groupe alkyle inférieur ou benzyle) avec une amine de formule générale (III)

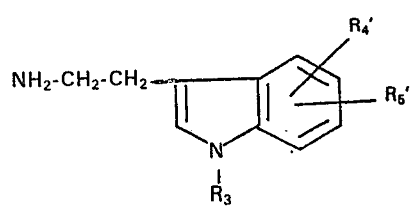

III

(dans laquelle $R_3$ a le sens précité et $R_4'$ et $R_5'$ ont les sens indiqués pour $R_4$ et $R_5$ à l'exclusion d'un groupe hydroxyle) et un solvant nucléophile de formule générale (IV):

$R_2OH$ IV

(dans laquelle $R_2$ a le sens précité) et, si on le désire, on soumet tous les groupes benzyloxy éventuellement présents dans les composés obtenus, de formule générale (Ia)

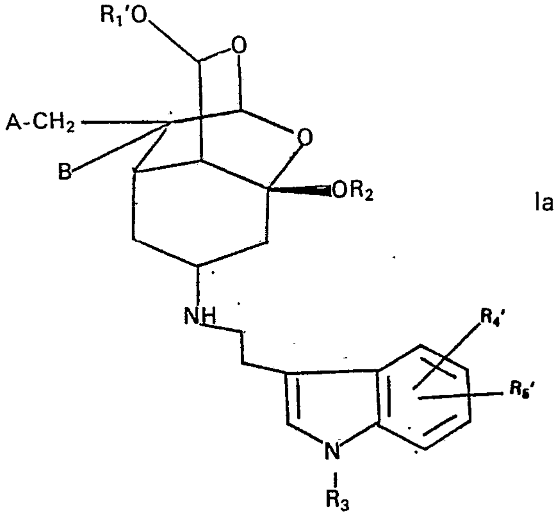

Ia

(dans laquelle A, B, $R_1'$, $R_2$, $R_3$, $R_4'$ et $R_5'$ ont le sens précité) à une hydrogénolyse donnant des groupes hydroxyles et/ou l'on transforme éventuellement des composés libres de formule (I) en leurs sels d'addition d'acides ou bien l'on transforme les sels d'addition d'acides en composés libres de formule (I).

7. Médicament contenant une quantité pharmacologiquement acitve d'un composé de formule (I) selon la revendication 1, dans lequel $R_1$ a le sens indiqué à la revendication 1 à l'exclusion d'un groupe benzyle, $R_4$ et $R_5$ ont le sens indiqué à la revendication 1 à l'exclusion du groupe benzyloxy, et $R_2$, $R_3$, A et B ont le sens indiqué à la revendication 1, et contenant des adjuvants et/ou excipients, véhicules ou supports usuels.

## Claims

1. Compounds of the general Formula (I)

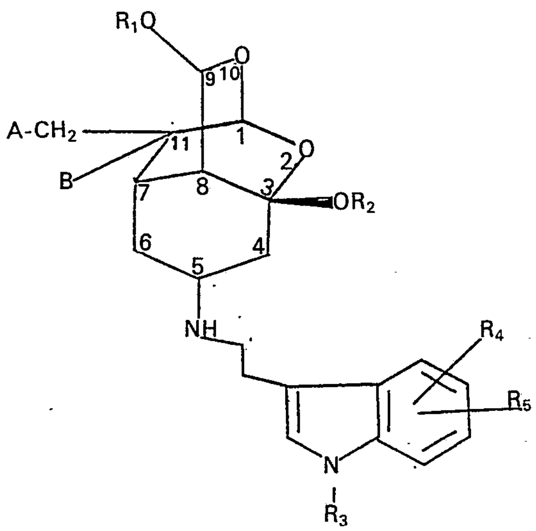

I

in which $R_1$ is hydrogen, alkyl with 1 to 4 carbon atoms or benzyl,

$R_2$ is alkyl with 1 to 4 carbon atoms or alkanoyl with 1 to 4 carbon atoms,

$R_3$ is hydrogen or alkyl with 1 to 4 carbon atoms,

$R_4$ is hydrogen, alkoxy with 1 to 4 carbon atoms, benzyloxy or hydroxy,

$R_5$ is hydrogen, alkoxy with 1 to 4 carbon atoms, benzyloxy or hydroxy, and

A and B are each hydrogen or together form a bond, and their acid addition salts.

2. Compounds of Formula (I) according to Claim 1, in which $R_1$ is alkyl with 1 to 4 carbon atoms.

3. Compounds of Formula (I) according to Claim 2, in which $R_3$ is hydrogen.

4. Compounds of Formula (I) according to Claim 3, in which $R_1$ and $R_2$ are alkyl with 1 to 4 carbon atoms and $R_3$, $R_4$ and $R_5$ are hydrogen.

5. Compounds of Formula (I) according to Claim 1, 2, 3 or 4, in which A and B together form a bond.

6. Method for the preparation of compounds of the general Formula (I)

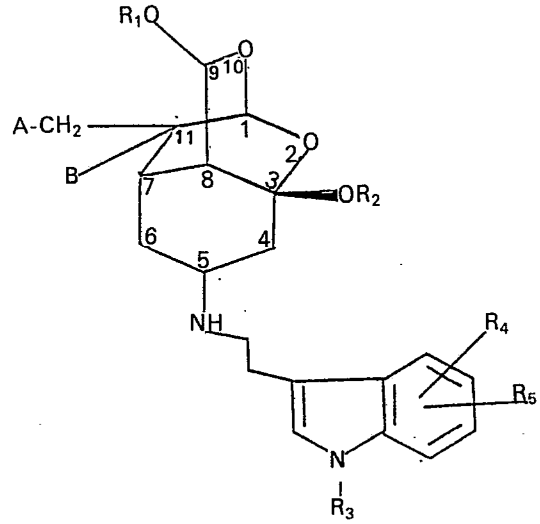

I

in which $R_1$, $R_2$, $R_3$, $R_4$, $R_5$, A and B have the meanings indicated in Claim 1, and their acid addition salts, characterised in that compounds of the general Formula (II)

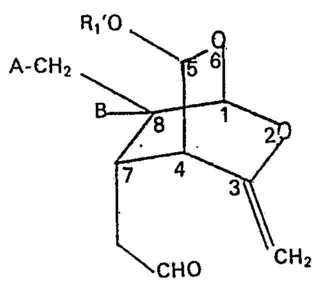

II

in which A and B have the above meanings and $R_1'$ is lower alkyl or benzyl, are reacted with an amine of the general Formula (III)

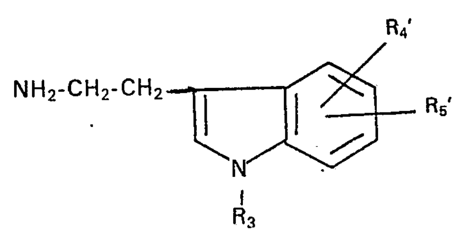

III

in which $R_3$ has the above meaning and $R_4'$ and $R_5'$ have the meanings given for $R_4$ and $R_5$ with the

exception of hydroxy, and with a nucleophilic solvent of the general Formula (IV)

$R_2OH$ IV

in which $R_2$ has the above meaning, and in the resulting compounds of the general Formula (Ia)

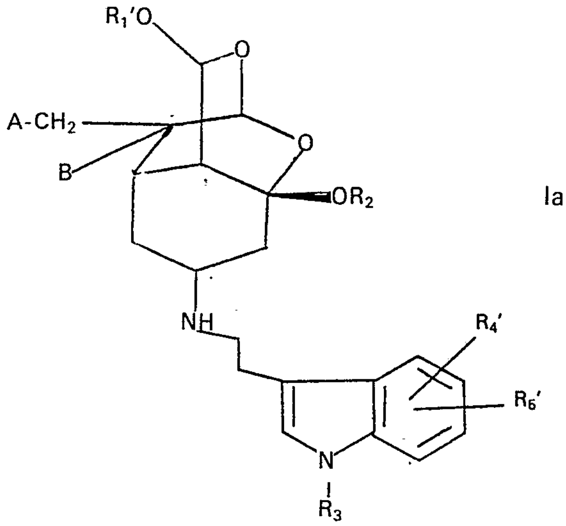

Ia

in which A, B, $R_1'$, $R_2$, $R_3$, $R_4'$ and $R_5'$ have the above meanings, if desired possible benzyloxy groups are split hydrogenolytically to hydroxy groups and/or optionally free compounds of the Formula (I) are converted into their acid addition salts or the acid addition salts are converted into the free compounds of Formula (I).

7. Medicament containing a pharmacologically effective quantity of a compound of Formula (I) according to Claim 1, in which $R_1$ has the meaning indicated in Claim 1 with the exception of benzyl, $R_4$ and $R_5$ have the meanings indicated in Claim 1 with the exception of benzyloxy and $R_2$, $R_3$, A and B have the meanings indicated in Claim 1, and conventional adjuvant and/or carrier substances.